# EUROPEAN PATENT SPECIFICATION

(11) **EP 1 267 884 B1**
(45) Date of publication and mention of the grant of the patent: **13.10.2004**
(21) Application number: 01933793.0
(22) Date of filing: 05.04.2001
(51) Int. Cl.: A61K 31/565, A61K 47/10, A61K 47/12, A61K 9/70

(54) **COMPOSITIONS FOR USE AS PENETRATION PROMOTERS IN TRANSDERMAL FORMULATIONS FOR HIGHLY LIPOPHILIC ACTIVE INGREDIENTS**
ZUBEREITUNGEN ZUR ANWENDUNG ALS PENETRATIONFÖRDERER IN TRANSDERMALEN ARZNEIMITTELN DIE HOCH-LIPOPHILE WIRKSTOFFE ENTHALTEN
COMPOSITIONS POUVANT ETRE UTILISEES COMME PROMOTEURS DE PENETRATION DANS DES FORMULATIONS TRANSDERMIQUES POUR DES INGREDIENTS A FORTE ACTIVITE LIPOPHILE

(30) Priority: 07.04.2000 DE 10019171
(43) Date of publication of application: 02.01.2003
(73) Proprietor: Schering Aktiengesellschaft, 13353 Berlin (DE)
(72) Inventor: LIPP, Ralph, 14169 Berlin (DE); FUNKE, Adrian, 10587 Berlin (DE); GÜNTHER, Clemens, 13357 Berlin (DE)
(86) International application number: PCT/EP2001/003896
(87) International publication number: WO 2001/076608

(56) References cited:
- EP-A- 0 569 338
- EP-A- 0 672 422
- WO-A-00/14104
- WO-A-93/14727
- WO-A-98/07740
- US-A- 4 956 171
- DATABASE WPI Section Ch, Week 199648 Derwent Publications Ltd., London, GB; Class A96, AN 1996-482103 XP002177400 & JP 08 245377 A (YAMANOUCHI PHARM CO LTD) , 24 September 1996 (1996-09-24)
- DATABASE WPI Section Ch, Week 199548 Derwent Publications Ltd., London, GB; Class B03, AN 1995-371108 XP002177401 & JP 07 252150 A (TTS GIJUTSU KENKYUSHO KK), 3 October 1995 (1995-10-03)
- DATABASE WPI Section Ch, Week 199306 Derwent Publications Ltd., London, GB; Class B02, AN 1993-049536 XP002177402 & JP 05 000946 A (NICHIBAN KK), 8 January 1993 (1993-01-08)

## Description

The invention relates to compositions for use as penetration promoters in transdermal formulations for highly lipophilic active ingredients.

Transdermal formulations are formulations which are applied to the surface of the body and deliver an active ingredient contained therein or an active ingredient combination through the barrier of the skin into the systemic circulation.

The term "transdermal formulation" is used for the purpose of the patent as generic term for all formulations for transdermal administration.

Transdermal formulations can in the simplest case be active ingredient solutions (transdermal solution formulations) which are applied to the skin. Transdermal formulations for the purpose of the patent are, for example, also active ingredient emulsions and suspensions, and ointments.

A particular form of transdermal formulations are transdermal systems. The term "transdermal system" is used for the purpose of the patent in a narrow definition for all transdermal plaster formulations.

Transdermal systems are normally divided into matrix transdermal systems and membrane transdermal systems.

Matrix transdermal systems consist in the simplest case of three layers arranged in parallel one on top of the other, namely a backing layer, a matrix and a detachable layer. The latter, which normally consists of plastic sheet or coated paper, is removed before the transdermal system is applied to the skin. The matrix contains the active ingredient to be administered and normally also has adhesive properties. If the matrix is insufficiently adhesive to adhere reliably to the area of skin to which the transdermal system is to be applied, it is also possible to provide an adhesive layer between matrix and detachable layer.

Membrane transdermal systems consist in the simplest case of four layers, namely a backing layer, a reservoir, a membrane and a detachable layer. The reservoir, which may contain active ingredients and excipients, is normally completely surrounded by backing layer and membrane. The ingredients from the reservoir can be released through the membrane. If the membrane is not intrinsically sufficiently adhesive to adhere to the area of skin to which the transdermal system is to be applied, it is also possible to provide an adhesive layer between membrane and detachable layer (at least in the edge region).

Transdermal formulations are preferably used for administration of active ingredients which, because of their physicochemical properties, are easily able to overcome the barrier of the skin. To do this, the active ingredients must have sufficient solubility both in the lipophilic stratum corneum and in the underlying hydrophilic living epidermis.

Flynn G., Stewart B. ( Drug Dev. Res. 13:169-185 (1988)) describe a good correlation between the in vitro partition coefficient (octanol/water) and skin permeability and recommend those active ingredients whose partition coefficient is about 100 (log P=2) as candidates for transdermal administration.

Guy et al.( Fundam. Appl. Toxicol. 17: 575-583(1991)) show a parabola-like dependence between the logarithm of the maximum penetration rate and the logarithm of the octanol/water partition coefficient with an apex maximum at log P=2.

More hydrophilic active ingredients can be administered by employing penetration promoters which facilitate passage through the (lipophilic) stratum comeum. It is possible to assess the efficacy of these penetration promoters by comparing the transdermal fluxes achievable using such formulations with fluxes through skin from which the stratum corneum has been completely removed, for example by repeated application and subsequent detachment of adhesive strips (called "tesa stripping").

Suhonen, T.M. et al. ( J. Contr. Rel. 59: 149-161 (1999)) provide an overview of the mode of action of various penetration promoters and suggest an effect on skin lipids for C₁₂ fatty acid derivatives and terpenes and an effect on skin proteins for 1,2-propanediol and dimethyl sulfoxide (DMSO). A linkage between these two penetration-enhancing principles is not suggested therein. DMSO is normally employed in concentrations of 2%, lauric acid in concentrations of 5 to 10%, and 1,2-propanediol in concentrations of 20 to 90%.

U.S. Patent 4,956,171 (Chang, Y.) describes the use of a dual system which consists of two penetration promoters with similar modes of action, namely sucrose cocoate and methyl laurate.

Cornwell, P.A. et al. ( Journal of Pharmacy and Pharmacology 46: 938 950 (1994)) describe a synergistic penetration-enhancing effect of 1,2-propanediol and terpenes.

Cooper E.R. ( J. Pharm. Sci. 73: 1153-1156 (1984)), describes an increase in the penetration of salicylic acid, an active ingredient of moderate lipophilicity, from 1,2-propanediol when fatty acids or fatty alcohols are added to the formulation.

PCT patent application WO 93/14727 describes transdermal systems with ion pairs from the basic active ingredient buprenorphine, and known fatty acids as penetration promoters.

Gao S., Singh J. ( J. Contr. Rel. 51: 193-199 (1998)) describe how it is possible to increase the permeability coefficient of the highly lipophilic basic antiestrogen active ingredient tamoxifen, on application of extremely dilute solutions, by a factor of 25 compared with water (or 40 compared with water/ethanol) by adding terpenes. However, the fluxes achieved are at least 5 orders of magnitude lower than necessary for therapy.

Gao S., Singh J. (Int. J. Pharm. 165: 45-55 (1998)) likewise show an increase in the permeability coefficient for oleic acid/ethanol and oleic acid/1,2-propanediol as penetration promoters, but by a factor of only 2.6 compared with phosphate buffer (or 6 compared with 1,2-propanediol alone). The fluxes achieved are likewise at least 5 orders of magnitude lower than necessary for therapy.

The route followed to date for transdermal administration of tamoxifen has therefore involved the use not of tamoxifen but of its active metabolite 4-hydroxytamoxifen. The introduction of the hydroxyl group reduces the lipophilicity of the molecule to such an extent that penetration through the skin is facilitated. Mauvais-Jarvis et al. ( Contracept Fertil Sex 19/2: 165-171 (1991)) propose transdermal administration onto the breast in order to achieve the same effect as with oral tamoxifen with a lower dosage and few systemic side effects. Pujol et al. ( Cancer Chemother. Pharmacol. 36(6): 493-498 (1995)) do not, however, achieve adequate transdermal fluxes in a phase 1 study.

DeGregorio et al. ( Cancer Chemother. Pharmacol. 39(6): 513-20 (1997)) describe an accumulation of the active ingredient toremifen which acts as an antiestrogen in the tumor after topical application of a gel.

The invention is therefore based on the object of developing transdermal formulations which make sufficiently high transdermal fluxes possible for active ingredients of high lipophilicity. For this purpose it was intended preferably to employ compositions of penetration promoters which, on the one hand, were to be well tolerated by skin and, on the other hand, were to be specifically suitable also for transdermal systems.

The object of the invention is achieved by compositions for use as penetration promoters in transdermal formulations composed of at least a first and a second penetration promoter, where the first penetration promoter is 1,2 propanediol, and the second penetration promoter is lauric acid.

The two penetration promoters are moreover preferably present in a ratio of from 2 to 15 parts by weight of first penetration promoter to 1 part by weight of second penetration promoter.

In this case, a ratio of about 2 to 9 parts by weight of 1,2-propanediol and about 1 part by weight of lauric acid has proved to be advantageous.

In solution formulations and membrane transdermal systems a ratio of about 9 parts by weight of 1,2-propanediol and about 1 part by weight of lauric acid, whereas in matrix transdermal systems a ratio of about 2 to 3 parts by weight of 1,2-propanediol and about 1 part by weight of lauric acid, is preferred.

The invention additionally relates to transdermal formulations, in particular transdermal systems for highly lipophilic active ingredients or active ingredient combinations, which comprise penetration-promoting compositions of the invention.

A total content of penetration-promoting composition in matrix transdermal systems of between 10 and 90% by weight is advantageous, preferably between 15 and 40% by weight and particularly preferably between 20 and 35% by weight.

A total content of penetration-promoting composition in a membrane transdermal system of between 50 and 100% by weight is advantageous, preferably between 80 and 100%.

The invention further relates to transdermal systems which comprise the compositions of the invention and in which the adhesive matrix is a polyacrylate, silicone or polyisobutylene adhesive.

Polyacrylates for the purpose of the patent is a generic term for all polymers (homopolymers and copolymers) which contain acrylic acid or acrylic acid derivatives.

Preference is given to vinyl acetate/acrylate copolymers and acrylate/vinyl-pyrrolidone copolymers, particularly preferably 2-ethylhexyl acrylate/N-vinyl-2-pyrrolidone.

The invention further relates to transdermal formulations, in particular transdermal systems, in which the active ingredient to be administered is a highly lipophilic active ingredient.

The highly lipophilic active ingredient is preferably basic and/or a steroid active ingredient. Highly lipophilic basic steroid active ingredients are particularly preferred. Most preference is given to highly lipophilic basic antiestrogens such as, for example, 11β-fluoro-7α-{5-[N-methyl-N-3-(4,4,5,5,5-pentafluoropentylthio)-propylamino]pentyl}estra-1,3,5(10)-triene-3,17β-diol (antiestrogen 1) or 11β-fluoro-7α-{5-[methyl-(7,7,8,8,9,9,10,10,10-nonafluorodecyl)amino]-pentyl}estra-1,3,5(10)-triene-3,17β-diol (antiestrogen 2).

It has been found, surprisingly, that the necessary increase in penetration of highly lipophilic active ingredients can be achieved by employing the compositions of the invention comprising at least two penetration promoters.

For the purposes of this invention, the term "highly lipophilic active ingredient" means that the active ingredient has an octanol/water partition coefficient of at least log P=3, preferably log P=4.

The efficacy of the novel transdermal formulations has been proved by way of example for the antiestrogen 1 (11β-fluoro-7α-{5-[N-methyl-N-3-(4,4,5,5,5-pentafluoropentylthio)propylamino]pentyl}estra-1,3,5(10)-triene-3,17β-diol) and the antiestrogen 2 (11β-fluoro-7α-{5-[methyl-(7,7,8,8,9,9,10,10,10-nonafluorodecyl)amino]pentyl}estra-1,3,5(10)-triene-3,17β-diol).

The former active ingredient has an octanol/water partition coefficient of log P=5.8 and is therefore to be classified as highly lipophilic. The second active ingredient has an octanol/water partition coefficient of log P=7.8 and therefore has an even greater lipophilicity.

The transdermal system of the invention is, however, of course not confined to these two very lipophilic active ingredients but can be employed with the same advantageous properties for all highly lipophilic active ingredients, for example for the aforementioned antiestrogens tamoxifen and toremifen, but also for substances with entirely different spectra of effects, for example highly lipophilic corticosteroids and highly lipophilic esters of corticosteroids, for example betamethasone valerate and clobetasone butyrate, other highly lipophilic sex steroids, for example desogestrel and mestranol, highly lipophilic esters of sex steroids, for example megestrol acetate and testosterone esters, highly lipophilic antimycotics, for example ketoconazole and itraconazole, highly lipophilic antihistamines, for example terfenadine, astemizole and cinnarizine, highly lipophilic antipsychotics, antidepressants and neuroleptics, for example chlorprothixen, flupenthixol, zuclopenthixol and haloperidol, highly lipophilic hypnotics, for example medazepam, highly lipophilic analgesics, for example diclofenac, highly lipophilic lipid-lowering agents, for example simvastatin, and highly lipophilic vitamins and derivatives thereof, for example retinol, retinol palmitate, tretinoin, calcitriol, tocopherol.

Lipinski et al. ( Adv. Drug Del. Rev. 23:3-25 (1997)) show that highly lipophilic drug substances are frequently found by the high throughput screening which is widely employed now, so that the possible uses of transdermal systems of the invention will be extended considerably more.

The penetration-promoting compositions of the invention can be incorporated into any conventional transdermal system. The penetration-promoting composition is moreover not confined to the two penetration promoters of the invention; on the contrary, it is, of course, additionally possible to include other penetration promoters.

It is also possible to employ other excipients customary for transdermal systems, such as, for example, crystallization inhibitors, anti-inflammatory agents and skin care agents, matrix-forming substances etc.

Although the particular penetration-enhancing properties of matrix transdermal systems and solution formulations are described in the following examples, similarly beneficial effects are to be expected when the compositions of the invention are incorporated into membrane transdermal systems.

For this purpose, an impermeable sheet is shaped by heat and/or tension so that one or more recesses holding 0.1 ml to 3 ml are produced. These are filled with a solution containing 0.5 to 50% by weight of active ingredient and 50 to 100% by weight of penetration-promoting composition, preferably containing 10% by weight of active ingredient and 90% by weight of penetration-promoting composition, preferably consisting of a mixture of 9 parts by weight of 1,2-propanediol and 1 part by weight of lauric acid. The active ingredient-containing solution can also be thickened with up to 10% by weight of matrix former. The reservoir is covered on the skin side by a welded-on or bonded-on permeable polymer layer onto which a permeable skin contact adhesive layer and a detachable protective layer are applied. The permeable polymer layer used is, for example, a sheet, 20 to 200 µm thick, of cellulose esters, cellulose ethers, silicones or polyolefin compounds. The rate of diffusion of the active ingredient and the penetration-promoting composition can be varied by variation in this polymer layer.

The materials suitable as adhesive and protective layer are the same as described for the matrix transdermal systems in Example 3.

Examples 1 and 2 show the penetration-enhancing properties of a composition of 9 parts by weight of 1,2-propanediol and 1 part by weight of lauric acid as solution formulation of antiestrogen 1. The transdermal fluxes achieved with this solution formulation are, on the one hand, a factor of at least 50 greater than on use of pure 1,2-propanediol or pure dimethylisosorbide (DMI) or a mixture of DMI and lauric acid and, on the other hand, about the same as after complete removal of the stratum comeum by "tesa stripping". The steady-state flux rates achieved absolutely are of the order of several µg/cm²/h. Therapeutic doses can therefore be administered - depending on the active ingredient, of course - straightforwardly with a plaster up to about 50 cm² in size.

The skin fluxes which can be achieved with the composition of the invention are moreover a factor of 100 larger than those of the DMI/DMSO/lauric acid solution formulation. This is surprising because it would have been possible to assume that the DMI/DMSO/lauric acid solution formulation would have a strong penetration-promoting effect because it - just like the formulation of the invention - acts at two different points (proteins and lipids) in the stratum corneum. This impressively proves the astonishingly strong synergistic effect of the composition of the invention, which evidently changes the structure of the stratum corneum so effectively that even the penetration of extremely lipophilic molecules is not impeded.

It has additionally been found that adequate steady-state flux rates can be achieved simply by pretreatment of the skin with a composition of the invention comprising the two penetration promoters from matrix transdermal systems which do not contain the compositions of the invention (see Example 3). The steady-state flux rates are of the order of up to 1 µg/cm²/h and are negligibly lower than that of the solution formulation. The concentration of the antiestrogen 1 (11β-fluoro-7α-{5-[N-methyl-N-3-(4,4,5,5,5-pentafluoropentylthio)-propylamino]pentyl}estra-1,3,5(10)-triene-3,17β-diol) in the adhesive matrix was between1 and 2 %. (m/m).
In this case, surprisingly, the fluxes from the most lipophilic of the adhesive matrices investigated, namely from the polyisobutylene matrix, were high.

It has additionally been found that the penetration promoter compositions of the invention can also be incorporated into plaster formulations. Example 4 shows such transdermal systems which contain the compositions of the invention and have an adhesive matrix composed of polyacrylic esters. The average skin fluxes achieved were somewhat lower than for the corresponding solution formulation.

Higher steady-state flux rates from the matrix transdermal systems containing the compositions of the invention can be achieved when the concentration of the active ingredient, specifically of the antiestrogen 1 (11β-fluoro-7α-{5-[N-methyl-N-3-(4,4,5,5,5-pentafluoropentylthio)-propylamino]pentyl}estra-1,3,5(10)-triene-3,17β-diol) is increased to 10% (m/m) and, at the same time, the skin is pretreated with the compositions of the invention.
The steady-state flux rates are of the order of up to 2.6 µg/cm²/h (see Example 5).

In summary, the invention relates to the following aspects:

One aspect of the invention relates to compositions for use as penetration promoters in transdermal formulations for highly lipophilic active ingredients or active ingredient combinations, which comprise at least a first and a second penetration promoter, where the first penetration promoter is 1,2 propanediol and the second penetration promoter is lauric acid.

The two penetration promoters are preferably present in a ratio of from 2 to 15 parts by weight of first penetration promoter to 1 part by weight of second penetration promoter.

The ratio preferred for compositions of 1,2-propanediol and lauric acid is about 2 to 9 parts by weight of 1,2-propanediol and about 1 part by weight of lauric acid. Particular preference is given to 2 to 3 and 9 parts by weight of 1,2-propanediol and about 1 part by weight of lauric acid.

A further aspect of the invention relates to transdermal formulations, specifically transdermal systems, which comprise the compositions described above. The transdermal systems of the invention may be matrix transdermal systems or membrane transdermal systems.

In matrix transdermal systems, the preferred total content of penetration-promoting composition in the matrix is between 10 and 90% by weight. The particularly preferred total content of penetration-promoting composition in the matrix is between 15 and 40% by weight, and a total content between 20 and 35% by weight is most preferred.

The adhesive matrix may be a polyacrylate adhesive, a vinyl acetate/acrylate copolymer adhesive an acrylate/vinylpyrrolidone copolymer adhesive, preferably 2-ethylhexyl acrylate/N-vinyl-2-pyrrollidone copolymer adhesive, a silicone adhesive or a polyisobutylene adhesive.

In membrane transdermal systems, the preferred total content of penetration-promoting composition in the reservoir is between 50 and 100% by weight. The particularly preferred total content of penetration-promoting composition in the reservoir is between 80 and 100% by weight.

The transdermal formulations of the invention, specifically the transdermal systems, are suitable for highly lipophilic active ingredients, in particular highly lipophilic basic active ingredients. They are particularly suitable for steroid active ingredients, in particular antiestrogens.
Most preference is given to the highly lipophilic basic antiestrogens 11β-fluoro-7α-{5-(N-methyl-N-3-(4,4,5,5,5-pentafluoropentylthio)propylamino]pentyl}-estra-1,3,5(10)-triene-3,17β-diol (antiestrogen 1) or 11β-fluoro-7α-{5-[methyl-(7,7,8,8,9,9,10,10,10-nonafluorodecyl)amino]pentyl}estra-1,3,5(10)-triene-3,17β-diol (antiestrogen 2).

### Examples

The skin fluxes stated in the following examples were determined by the following in vitro diffusion test:
A temperature-controlled flow cell is divided by a piece, 2 cm² in size, of excised skin from nude mice into a donor compartment and an acceptor compartment, the stratum corneum facing the donor side. A 3% strength or 5% strength solution of hydroxypropyl-β-cyclodextrin in buffer is pumped by a pneumatic pump from a temperature-controlled reservoir through the acceptor compartment and is collected with the aid of a fraction collector in glass vials which are replaced at defined time intervals. On the donor side, solutions containing excipients and/or active ingredients are applied or plasters containing excipients and/or active ingredients are stuck on. The content of active ingredients is determined in the individual fractions by HPLC/UV. The cumulative absorbed dose is plotted against time. The gradient of the linear part of the plot is taken as the average steady-state flux through the skin.

### Example 1

### Solution formulations

### a) Solution formulations with antiestrogen 1

In each case 2% strength solution formulations of antiestrogen 1 (11β-fluoro-7α-{5-[N-methyl-N-3-(4,4,5,5,5-pentafluoropentylthio)propylamino]pentyl}-estra-1,3,5(10)-triene-3,17β-diol) in various compositions of penetration promoters as shown in Table la are produced. The solution formulations are applied to mouse skin as described above (20 µl per 2 cm² area of skin). The following skin fluxes are achieved in the in vitro diffusion test:

**Table Ia**

| Composition of penetration promoter *(parts by weight)* | Average flux *[µg*/*cm*^{*2*}/*h]* | No. of cases |
|---|---|---|
| Dimethylisosorbide (pure) | 0.03 | n = 9 |
| Dimethylisosorbide/lauryl alcohol 95 + 5 | 0.07 | n = 2 |
| Dimethylisosorbide/lauric acid 9 + 1 | 0.07 | n = 3 |
| Dimethylisosorbide/DMSO/lauric acid 88 + 2 + 10 | 0.015 | n = 3 |
| 1,2-Propanediol (pure) | 0.045 | n = 6 |
| 1,2-Propanediol/DMSO 98 + 2 | 0.09 | n = 3 |
| 1,2-Propanediol/lauric acid 9 + 1 | 5.8 | n = 8 |

### b) Solution formulations with antiestrogen 2

In each case 2% strength solution formulations of antiestrogen 2 (11β-fluoro-7α-{5-[methyl-(7,7,8,8,9,9,10,10,10-nonafluorodecyl)amino]pentyl}estra-1,3,5(10)-triene-3,17β-diol) in various compositions of penetration promoters as shown in Table Ib are produced. The solution formulations are applied to mouse skin as described above (20 µl per 2 cm² area of skin). The following skin fluxes are achieved in the in vitro diffusion test:

**Table Ib**

| Composition of penetration promoter *(parts by weight)* | Average flux *[µg*/*cm*^{*2*}/*h]* | No. of cases |
|---|---|---|
| 1,2-Propanediol (pure) | < 0.01 (not detectable) | n = 3 |
| 1,2-Propanediol/lauric acid 9 + 1 | 3.2 | n = 3 |

### Example 2

### Skin flux after removal of the stratum corneum

The stratum comeum was removed from the mouse skin by "tesa stripping" before clamping in the flow cell. A 2% strength solution of antiestrogen 1 (11β-fluoro-7α-{5-[N-methyl-N-3-(4,4,5,5,5-pentafluoropentylthio)propylamino]-pentyl}estra-1,3,5(10)-triene-3,17β-diol) in 1,2-propanediol was prepared as described in Example 1, and 20 µl of this solution formulation was applied to the mouse skin treated in this way (skin area 2 cm²).
The following skin fluxes are achieved in the in vitro diffusion test:

**Table II**

| Composition of penetration promoter *(parts by weight)* | Average flux *[µg*/*cm*^{*2*}/*h]* | No. of cases |
|---|---|---|
| 1,2-Propanediol (pure) | 6.3 | n = 3 |

### Example 3:

### Pretreatment of the skin with the compositions of the invention

### (1) Preparation of the matrix transdermal systems

Various active ingredient-containing matrix transdermal systems (A-D) which do not contain the compositions of the invention are prepared:
A. A solution of 0.2 g of antiestrogen 1 (11β-fluoro-7α-{5-[N-methyl-N-3-(4,4,5,5,5-pentafluoropentylthio)propylamino]pentyl}estra-1,3,5(10)-triene-3,17β-diol) in 6.9 g of ethyl acetate is introduced into 19.6 g of a 50% strength solution of a vinyl acetate/acrylate copolymer (e.g. Gelva Multipolymer Solution type 2723 or 7883 from Solutia, formerly Monsanto) in ethyl acetate. This mixture is stirred with a magnetic stirrer for 15 minutes until dissolution is complete.
B. A solution of 0.2 g of antiestrogen 1 in 8.7 g of ethyl acetate is introduced into 27.45 g of a 35.7% strength solution of 2-ethylhexyl acrylate/N-vinyl-2-pyrollidone copolymer (from Sekisui) in ethyl acetate. This mixture is stirred with a magnetic stirrer for 15 minutes until dissolution is complete.
C. A solution of 0.2 g of antiestrogen 1 in 3.8 g of ethyl acetate is introduced into 15.64 g of a 60% strength solution of silicones (e.g. Bio-PSA type X7-4502 from Dow-Corning) in ethyl acetate. 0.83 g of hexane is then added. This mixture is stirred with a magnetic stirrer for 15 minutes until dissolution is complete.
D. A solution of 0.2 g of antiestrogen 1 in 15 g of ethyl acetate is introduced into 20.1 g of a 48.76% strength solution of polyisobutylenes (e.g. Oppanols type B12SF from BASF) in hexane. This mixture is stirred with a magnetic stirrer for 15 minutes until dissolution is complete.

Using a coating device (400 µm knife), these solutions are applied as uniform films to a fluoropolymer-coated detachable layer, e.g. Scotchpak 1022 from 3M. Ethyl acetate and hexane are completely removed at 70°C. This is followed by lamination with a backing layer, e.g. CoTran polyethylene film from 3M. The laminate obtained in this way is divided by means of a punch device into circular individual plasters and packed in aluminum foil.
The result is plasters which are microscopically free of crystals and have suitable adhesive properties and have active ingredient contents of about 1 to 2% (m/m) based on the matrix weight as shown in Table IIIa (column 2).

### (2) Pretreatment of the skin with the compositions of the invention

After a pretreatment of the skin with a composition of the invention, the prepared matrix transdermal systems are applied, and the skin fluxes are measured.

The procedure involves pretreatment of the areas of skin for 16 hours with 20 µl portions of a 10% strength (m/m) solution of lauric acid in propanediol (1+9). Then plasters 2 cm² in size are, after detachment of the liner, stuck into mouse skin (skin areas 2 cm² in size).
The following skin fluxes are achieved in the in vitro diffusion test:

**Table III**

| Plaster adhesive system | Active ingredient content | Average flux *[µg*/*cm*^{*2*}/*h]* | Number of cases |
|---|---|---|---|
| A. Vinyl acetate/acrylate copolymer (Gelva) | 1.8% | 1.08 | n = 3 |
| B. 2-Ethylhexyl acrylate/N-vinyl-2-pyrrolidone copolymer (Sekisui) | 1.5% | 0.12 | n = 3 |
| C. Silicone | 1.6% | 0.31 | n = 3 |
| D. Polyisobutylene | 1.3% | 0.55 | n = 3 |

### Example 4

Matrix transdermal system containing composition of the invention.

This example relates to the preparation of 6 different types of plaster with the penetration promoter composition of the invention by the general method:

### (1) Preparation of the matrix transdermal systems

3.5 g of propanediol and a solution of lauric acid (weight shown in Table IVa) and antiestrogen 1 (11β-fluoro-7α-{5-[N-methyl-N-3-(4,4,5,5,5-pentafluoropentylthio)propylamino]pentyl}estra-1,3,5(10)-triene-3,17β-diol) (weight shown in Table IVa) in 3.5 g of ethyl acetate are successively introduced into a 50% strength solution of a vinyl acetate/acrylate copolymer (e.g. Gelva Multipolymer Solution type 2723 or 7883 from Solutia, formerly Monsanto) in ethyl acetate (weight varies as shown in Table IVa).

This mixture is made up to 20 g with ethyl acetate and stirred with a magnetic stirrer for 15 minutes until dissolution is complete.

**Table IVa**

| Transdermal system | Polymer solution *[g]* | Lauric acid *[g]* | Active ingredient *[g]* |
|---|---|---|---|
| 1 | 12.2 | 0.2 | 0.2 |
| 2 | 11.6 | 0.5 | 0.2 |
| 3 | 10.6 | 1.0 | 0.2 |
| 4 | 11.2 | 0.5 | 0.4 |
| 5 | 10.4 | 0.5 | 0.8 |
| 6 | 8.8 | 0.5 | 1.6 |

Using a coating device (400 µm knife), the mixture is applied as a uniform film to a fluoropolymer-coated detachable layer. At 70°C, ethyl acetate is completely removed, and 1,2-propanediol is removed to a desired residual content of 20% of the matrix weight. This is followed by lamination with a backing layer. The laminate obtained in this way is divided by a punch device into circular individual plasters and packed in aluminum foil. The result is plasters which are microscopically free of crystals and have suitable adhesive properties and have active ingredient and excipient contents based on the matrix weight (m/m) as shown in Table IVb.

**Table IVb**

| Transdermal system | 1,2-Propanediol *[g]* | Lauric acid *[g]* | Active ingredient *[g]* |
|---|---|---|---|
| 1 | 21.0 | 2.4 | 2.4 |
| 2 | 23.7 | 6.4 | 2.5 |
| 3 | 26.8 | 11.2 | 2.3 |
| 4 | 17.0 | 6.0 | 4.5 |
| 5 | 19.7 | 5.8 | 9.7 |
| 6 | 20.8 | 6.2 | 18.9 |

### (2) Determination of the skin fluxes

The plasters 2 cm² in size are, after detachment of the liner, stuck onto mouse skin.

The following skin fluxes are achieved in the in vitro diffusion test:

**Table IVc**

| Transdermal system | Average flux *[µg*/*cm*^{*2*}/*h]* | Number of cases |
|---|---|---|
| 1 | 0.066 | n = 3 |
| 2 | 0.045 | n = 6 |
| 3 | 0.092 | n = 3 |
| 4 | 0.120 | n = 3 |
| 5 | 0.141 | n = 3 |
| 6 | 0.107 | n = 3 |

The same tests were also carried out with antiestrogen 2 (11β-fluoro-7α-{5-[methyl-(7,7,8,8,9,9,10,10,10-nonafluorodecyl)amino]pentyl}estra-1,3,5(10)-triene-3,17β-diol) and led to similar results.

### Example 5

Pretreatment of the skin with the compositions of the invention and matrix transdermal system containing composition of the invention

### (1) Preparation of the matrix transdermal systems of the invention

A solution of 1 g of antiestrogen 1 (11β-fluoro-7α-{5-[N-methyl-N-3-(4,4,5,5,5-pentafluoropentylthio)propylamino]pentyl}estra-1,3,5(10)-triene-3,17β-diol), 1 g of lauric acid and 0.4 g of hydroxypropylcellulose 75 000 in 7.1 g of ethyl acetate is introduced into 7.2 g of a 50% strength solution of vinyl acetate/acrylate copolymer (e.g. Gelva Multipolymer Solution type 2723 or 7883 from Solutia, formerly Monsanto) in ethyl acetate. This mixture is stirred with a magnetic stirrer until dissolution is complete. Then 4 g of 1,2-propanediol are added and processed to a clearly transparent mixture.

Using a coating device (400 µm knife), the mixture is applied as uniform film to a fluoropolymer-coated detachable layer, e.g. Scotchpak 1022 from 3M. At 70°C, ethyl acetate is completely removed, and 1,2-propanediol is removed to a desired remaining content of about 30% of the matrix weight. This is followed by lamination with a backing layer, e.g. CoTran polyethylene film from 3M. The laminate obtained in this way is divided by means of a punch device into circular individual plasters and packed in aluminum foil. The result is plasters which are microscopically free of crystals and have suitable adhesive properties and contain 10.4% (m/m) antiestrogen 1, 10.1% lauric acid and 31.3% 1,2-propanediol based on the matrix weight.

### (2) Pretreatment of the skin with the composition of the invention

To carry out the diffusion test described above, the skin areas are pretreated with 20 µl portions of a 10% strength (m/m) solution of lauric acid in 1,2-propanediol (1 + 9) for 16 hours. The plasters, 2 cm² in size are, after detaching the liner, stuck onto mouse skin. The following skin fluxes are achieved in the in vitro diffusion test:

**Table V**

| Transdermal system | Average flux *[µg*/*cm*^{*2*}/*h]* | Number of cases |
|---|---|---|
| 1,2-Propanediol + lauric acid 3:1 (adhesive matrix: vinyl acetate/acrylate copolymer) | 2.6 | n = 3 |

The features of the invention in the above description and disclosed in the claims may be essential both singly and in any combination for implementing the invention in its various embodiments.

## Claims

1. A transdermal formulation comprising a highly lipophilic active ingredient or active ingredient combination with a log P ≥ 4 and a penetration promoting composition, which comprises at least a first and a second penetration promoter, where the first penetration promotor is 1,2-propanediol, and the second penetration promoter is lauric acid and wherein the two penetration promoters are present in a ratio of from 2 to 15 parts by weight of first penetration promoter to 1 part by weight of second penetration promoter.

2. A formulation as claimed in claim 1, having a ratio of about 2 to 9 parts by weight of 1,2-propanediol and about 1 part by weight of lauric acid.

3. A transdermal formulation as claimed in any of preceding claims, which is a transdermal system.

4. A transdermal system as set forth in claim 3, which is a matrix transdermal system.

5. A transdermal system as claimed in claim 4, wherein the total content of penetration-promoting composition in the matrix is between 10 and 90% by weight.

6. A transdermal system as set forth or claimed in any of claims 3 to 5, wherein the adhesive matrix is a polyacrylate adhesive, a vinyl acetate/acrylate copolymer adhesive, an acrylate/vinylpyrrolidone copolymer adhesive, preferably 2-ethylhexyl acrylate/N-vinyl-2-pyrrolidone copolymer adhesive, a silicone adhesive or a polyisobutylene adhesive.

7. A transdermal system as set forth in claim 3, which is a membrane transdermal system.

8. A transdermal system as claimed in claim 7, wherein the total content of penetration-promoting composition in the reservoir is between 50 and 100% by weight.

9. A transdermal formulation as claimed in claim 1, wherein the active ingredient to be administered is a basic, active ingredient.

10. A transdermal formulation as claimed in claim 9, wherein the active ingredient to be administered is a steroid active ingredient.

11. A transdermal formulation comprising an antiestrogen and a penetration promoting composition, which comprises at least a first and a second penetration promoter, where the first penetration promotor is 1,2-propanediol, and the second penetration promoter is lauric acid and wherein the two penetration promoters are present in a ratio of from 2 to 15 parts by weight of first penetration promoter to 1 part by weight of second penetration promoter.

12. A transdermal formulation as claimed in claim 11, wherein the active ingredient to be administered is 11β-fluoro-7α-{5-[N-methyl-N-3-(4,4,5,5,5-pentafluoropentylthio)propylamino]pentyl}estra-1,3,5(10)-triene-3,17β-diol (antiestrogen 1) or 11β-fluoro-7α-{5-[methyl-(7,7,8,8,9,9,10,10,10-nonafluorodecyl)amino]pentyl}estra-1,3,5(10)-triene-3,17β-diol (antiestrogen 2).

## Patentansprüche

1. Transdermale Formulierung, umfassend einen hoch lipophilen Wirkstoff oder eine hoch lipophile Wirkstoffkombination mit log P ≥ 4 und eine penetrationsverstärkende Zusammensetzung, umfassend einen ersten und einen zweiten Penetrationsverstärker, wobei der erste Penetrationsverstärker 1,2-Propandiol ist und der zweite Penetrationsverstärker Laurinsäure ist und wobei die beiden Penetrationsverstärker in einem Verhältnis von 2 bis 15 Gewichtsteilen erster Penetrationsverstärker zu einem Gewichtsteil zweiter Penetrationsverstärker enthalten sind.

2. Formulierung nach Anspruch 1, **gekennzeichnet durch** ein Verhältnis von etwa 2 bis 9 Gewichsteilen 1,2-Propandiol und etwa 1 Gewichtsteil Laurinsäure.

3. Transdermale Formulierung nach einem der vorhergehenden Ansprüche, welche ein Transdermalsystem ist.

4. Transdermalsystem nach Anspruch 3, welches ein Matrix-Transdermalsystem ist.

5. Transdermalsystem nach Anspruch 4, bei dem der Gesamtgehalt an penetrationsverstärkender Zusammensetzung in der Matrix zwischen 10 und 90 Gew.-% liegt.

6. Transdermalsystem nach einem der Ansprüche 3 bis 5, **dadurch gekennzeichnet, daß** die Klebstoffmatrix ein Polyacrylat-Kleber, ein Vinylacetat-Acrylat-Copolymer-Kleber, ein Acrylat-Vinyl-pyrrolidon-Copolymer-Kleber, vorzugsweise 2-Ethylhexylacrylat-N-vinyl-2-pyrrolidon-Copolymer-Kleber, ein Silikon-Kleber oder ein Polyisobutylen-Kleber ist.

7. Transdermalsystem nach Anspruch 3, **dadurch gekennzeichnet, daß** es ein Membran-Transdermalsystem ist.

8. Transdermalsystem nach Anspruch 7, bei dem der Gesamtgehalt an penetrationsverstärkender Zusammensetzung im Reservoir zwischen 50 und 100 Gew.-% liegt.

9. Transdermale Formulierung gemäß Anspruch 1, bei der der zu applizierende Wirkstoff ein basischer Wirkstoff ist.

10. Transdermale Formulierung gemäß Anspsruch 9, bei der der zu applizierende Wirkstoff ein Steroid-Wirkstoff ist.

11. Transdermale Formulierung, umfassend ein Antiestrogen und eine penetrationsverstärkende Zusammensetzung, umfassend mindestens einen ersten und einen zweiten Penetrationsverstärker, wobei der erste Penetrationsverstärker 1,2-Propandiol ist und der zweite Penetrationsverstärker Laurinsäure ist und wobei die beiden Penetrationsverstärker in einem Verhältnis von 2 bis 15 Gewichtsteilen erster Penetrationsverstärker zu einem Gewichtsteil zweiter Penetrationsverstärker enthalten sind.

12. Transdermale Formulierung gemäß Anspruch 11, bei der der zu applizierende Wirkstoff 11β-Fluor-7α-{5-[N-methyl-N-3-(4,4,5,5,5-pentafluorpentylthio)-propylamino]-pentyl}estra-1,3,5(10)-trien-3,17β-diol (Antiestrogen 1) oder 11β-Fluor-7α-{5-[methyl-(7,7,8,8,9,9,10,10,10-nonafluor-decyl)-amino]-pentyl}estra-1,3,5(10)-trien-3,17β-diol (Antiestrogen 2)ist.

## Revendications

1. Préparation transdermique comprenant un composant actif hautement lipophile ou une association hautement lipophile de composants actifs, ayant une valeur log P ≥ 4 et une composition facilitant la pénétration comprenant au moins un premier promoteur de pénétration et un second, le premier promoteur de pénétration étant le 1,2-propanediol et le second promoteur de pénétration étant l'acide laurique, et dans laquelle les deux promoteurs de pénétration sont présents en un rapport de 2 à 15 parties en poids du premier promoteur de pénétration à 1 partie en poids du second promoteur de pénétration.

2. Préparation telle que revendiquée dans la revendication 1, ayant un rapport d'environ 2 à 9 parties en poids de 1,2-propanediol pour environ 4 parties en poids d'acide laurique.

3. Préparation transdermique telle que revendiquée dans l'une quelconque des revendications précédentes, qui est un système transdermique.

4. Système transdermique tel qu'énoncé dans la revendication 3, qui est un système transdermique matriciel.

5. Système transdermique tel que revendiqué dans la revendication 4, dans lequel la concentration totale de la composition facilitant la pénétration dans la matrice est comprise entre 10 et 90 % en poids.

6. Système transdermique tel qu'énoncé ou revendiqué dans l'une quelconque des revendications 3 à 5, dans lequel la matrice adhésive est un adhésif polyacrylate, un adhésif copolymère acétate de vinyle/acrylate, un adhésif copolymère acrylate/vinylpyrrolidone, de préférence un adhésif copolymère acrylate de 2-éthylhexyle/N-vinyl-2-pyrrolidone, un adhésif silicone ou un adhésif polyisobutylène.

7. Système transdermique tel qu'énoncé dans la revendication 3, qui est un système transdermique à membrane.

8. Système transdermique tel que revendiqué dans la revendication 7, dans lequel la concentration totale de la composition facilitant la pénétration, dans le réservoir, est comprise entre 50 et 100 % en poids.

9. Préparation transdermique telle que revendiquée dans la revendication 1, dans laquelle le composant actif à administrer est un composant actif basique.

10. Préparation transdermique telle que revendiquée dans la revendication 9, dans laquelle le composant actif à administrer est un composant actif stéroïdien.

11. Préparation transdermique comprenant un anti-oestrogène et une composition facilitant la pénétration, comprenant au moins un premier promoteur de pénétration et un second, le premier promoteur de pénétration étant le 1,2-propanediol et le second promoteur de pénétration étant l'acide laurique, et dans laquelle les deux promoteurs de pénétration sont présents en un rapport de 2 à 15 parties en poids du premier promoteur de pénétration à 1 partie en poids du second promoteur de pénétration.

12. Préparation transdermique telle que revendiquée dans la revendication 11, dans laquelle le composant actif à administrer est le 11β-fluoro-7α-{5-[N-méthyl-N-3-(4,4,5,5,5-pentafluoropentylthio)propylamino]pentyl}-estra-1,3,5(10)-triène-3,17β-diol (anti-oestrogène 1) ou le 11β-fluoro-7α-{5-[méthyl-(7,7,8,8,9,9,10,10,10-nonafluorodécyl)-amino]pentyl}-estra-1,3,5(10)-triène-3,17β-diol (anti-oestrogène 2).
